# EUROPEAN PATENT APPLICATION

(11) **EP 3 922 198 A1**
(43) Date of publication of application: **15.12.2021**
(21) Application number: 21175770.3
(22) Date of filing: 25.05.2021
(51) Int. Cl.: A61B 17/34

(54) **SURGICAL ACCESS ASSEMBLY WITH RELEASE FEATURE**

(30) Priority: 08.06.2020 US 202016895140
(71) Applicant: Covidien LP, Mansfield, MA 02048 (US)
(72) Inventor: Desjardin, Kevin, Prospect, 06712 (US); Lobo, Astley C., 06516, West Haven (US); Pattison, Douglas M., Bristol, 06108 (US); Tokarz, Christopher A., Wallingford, 06492 (US); Baril, Jacob C., Norwalk, 06851 (US)
(74) Representative: Maschio & Soames IP Ltd

(57) **Abstract**

A surgical access assembly includes a surgical access device having a cannula tube, an expandable balloon positioned in a distal region of the cannula tube, and an inflation assembly positioned in a proximal region of the cannula tube. The inflation assembly has a port and a check valve. The port is in fluid communication with the expandable balloon and the check valve is transitionable between rest and actuated configurations. A syringe includes a barrel with a chamber and a plunger slidably disposed in the chamber. An extension of the barrel is configured for releasably engaging the check valve. The plunger also includes a release feature that is engageable with the check valve to transition the check valve from the rest configuration to the actuated configuration.

## Description

### FIELD

The present disclosure generally relates to a surgical access assembly. In particular, the present disclosure relates to a surgical access assembly having a release feature for actuating a check valve of a surgical access device.

### BACKGROUND

In minimally invasive surgical procedures, including endoscopic and laparoscopic surgeries, a surgical access device permits the introduction of a variety of surgical instruments into a body cavity or opening. A surgical access device (e.g., a cannula or an access port) is introduced through an opening in tissue (e.g., a naturally occurring orifice or an incision) to provide access to an underlying surgical site in the body. The opening is typically made using an obturator having a blunt or sharp tip that may be inserted through a passageway of the surgical access device. For example, a cannula has a tube of rigid material with a thin wall construction, through which an obturator may be passed. The obturator is utilized to penetrate a body wall, such as an abdominal wall, or to introduce the surgical access device through the body wall, and is then removed to permit introduction of surgical instruments through the surgical access device to perform the minimally invasive surgical procedure.

Minimally invasive surgical procedures, including both endoscopic and laparoscopic procedures, permit surgery to be performed on organs, tissues, and vessels far removed from an opening within the tissue. In laparoscopic procedures, the abdominal cavity is insufflated with an insufflation gas, e.g., CO₂, to create a pneumoperitoneum thereby providing access to the underlying organs. A laparoscopic instrument is introduced through a cannula into the abdominal cavity to perform one or more surgical tasks. The cannula may incorporate a seal to establish a substantially fluid tight seal about the laparoscopic instrument to preserve the integrity of the pneumoperitoneum. The cannula, which is subjected to the pressurized environment, e.g., the pneumoperitoneum, may include an anchor to prevent the cannula from backing out of the opening in the abdominal wall, for example, during withdrawal of the laparoscopic instrument from the cannula.

### SUMMARY

A surgical access assembly includes a surgical access device with a cannula tube, an expandable balloon positioned in a distal region of the cannula tube, and an inflation assembly positioned in a proximal region of the cannula tube. The inflation assembly includes a port and a check valve. The port is in fluid communication with the expandable balloon and the check valve is transitionable between a rest configuration and an actuated configuration. The rest configuration allows fluid flow in a first direction and inhibits fluid flow in a second direction opposite the first direction. The actuated configuration allows fluid flow in the first and second directions. The surgical access assembly also includes a syringe having a barrel with proximal and distal openings defining a chamber therebetween that is configured to store inflation fluid therein. An extension of the syringe is configured to releasably engage a portion of the check valve. The syringe also has a plunger that is slidably received in the chamber through the proximal opening and is configured to discharge at least a portion of the inflation fluid stored therein through the distal opening. The plunger also includes a release feature engageable with the check valve to transition the check valve from the rest configuration to the actuated configuration.

In aspects, the release feature may be insertable into the port to transition the check valve from the rest configuration to the actuated configuration.

In one aspect, the distal opening of the barrel may be engaged with the port such that translation of the plunger into the chamber delivers inflation fluid through the port thereby transitioning the check valve from the rest configuration to the actuated configuration.

In another aspect, the release feature may be a protrusion located on a distal end of the plunger.

In an aspect, the expandable balloon may transition from a collapsed configuration to an expanded configuration as the plunger translates distally through the chamber.

In aspects, the check valve may include a piston and a spring that biases the piston proximally towards the port.

In an aspect, the protrusion may be engageable with a portion of the piston and adapted to translate the piston distally thereby transitioning the check valve from the rest configuration to the actuated configuration.

A method of accessing a surgical site includes inserting a portion of a surgical access device into a patient. The surgical access device includes a cannula tube, an expandable balloon disposed in a distal region of the cannula tube, and an inflation assembly disposed in a proximal region of the cannula tube. The method also includes coupling a syringe to the inflation assembly such that a distal opening of a barrel of the syringe is positioned in a port of the inflation assembly. Additionally, the method includes translating a plunger in a chamber of the barrel thereby pressurizing inflation fluid in the chamber and transitioning a check valve of the inflation assembly from a rest configuration to an actuated configuration such that the chamber is in fluid communication with the expandable balloon. Further, the method includes transitioning the expandable balloon from a collapsed configuration to an expanded configuration by continued translation of the plunger towards a distal region of the chamber such that the check valve remains in the actuated configuration and inflation fluid is transferred from the chamber to the expandable balloon.

In an aspect, the method may include uncoupling the syringe from the inflation assembly, inserting a surgical instrument through the cannula tube, and performing a surgical procedure at the surgical site.

In another aspect, the method may include inserting a protrusion of the plunger into the port thereby transitioning the check valve from the rest configuration to the actuated configuration.

In aspects, the method may include removing the surgical access device from the patient and transitioning the expandable balloon from the expanded configuration to the collapsed configuration by venting the inflation fluid through the check valve and the port.

Other features of the disclosure will be appreciated from the following description.

### DESCRIPTION OF THE DRAWINGS

The accompanying drawings, which are incorporated in and constitute a part of this specification, illustrate aspects and features of the disclosure and, together with the detailed description below, serve to further explain the disclosure, in which:
FIG. 1 is a perspective view of a surgical access assembly with a surgical access device and a syringe;
FIG. 2 is a perspective view of the syringe of FIG. 1;
FIG. 3 is an exploded perspective view, with parts separated, of the syringe of FIG. 2;
FIG. 4 is a side cross-sectional view of the surgical access assembly of FIG. 1 taken along section line 4-4;
FIG. 4A is a cross-sectional view of the surgical access assembly of FIG. 1 taken along section line 4A-4A;
FIG. 5 is an enlarged view of the area of detail of FIG. 4 showing a check valve in an actuated configuration;
FIG. 6 is the enlarged view of FIG. 5 showing the check valve in a rest configuration;
FIG. 7 is a perspective view of the surgical access assembly of FIG. 1 with a plunger of the syringe coupled to an inflation assembly of the surgical access device;
FIG. 8 is a side cross-sectional view of the surgical access assembly of FIG. 7 taken along section line 8-8 and partially inserted into tissue;
FIG. 9 is an enlarged view of the area of detail of FIG. 8 showing engagement between the check valve and a release feature; and
FIG. 10 is the side cross-sectional view of the surgical access assembly of FIG. 8 removed from tissue.

### DETAILED DESCRIPTION

Aspects of the disclosure are described hereinbelow with reference to the accompanying drawings; however, it is to be understood that the disclosed aspects are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

Descriptions of technical features of an aspect of the disclosure should typically be considered as available and applicable to other similar features of another aspect of the disclosure. Accordingly, technical features described herein according to one aspect of the disclosure may be applicable to other aspects of the disclosure, and thus duplicative descriptions may be omitted herein. Like reference numerals may refer to like elements throughout the specification and drawings.

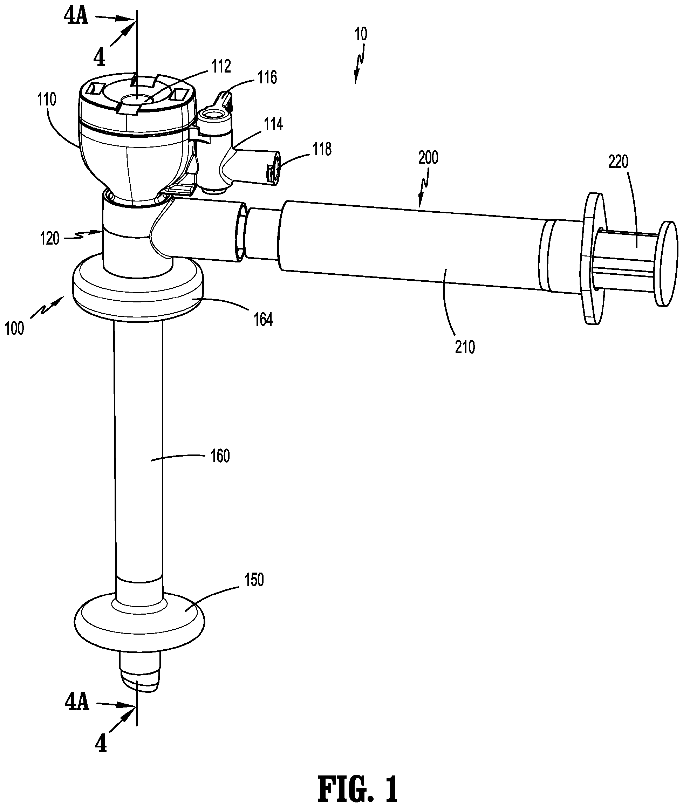

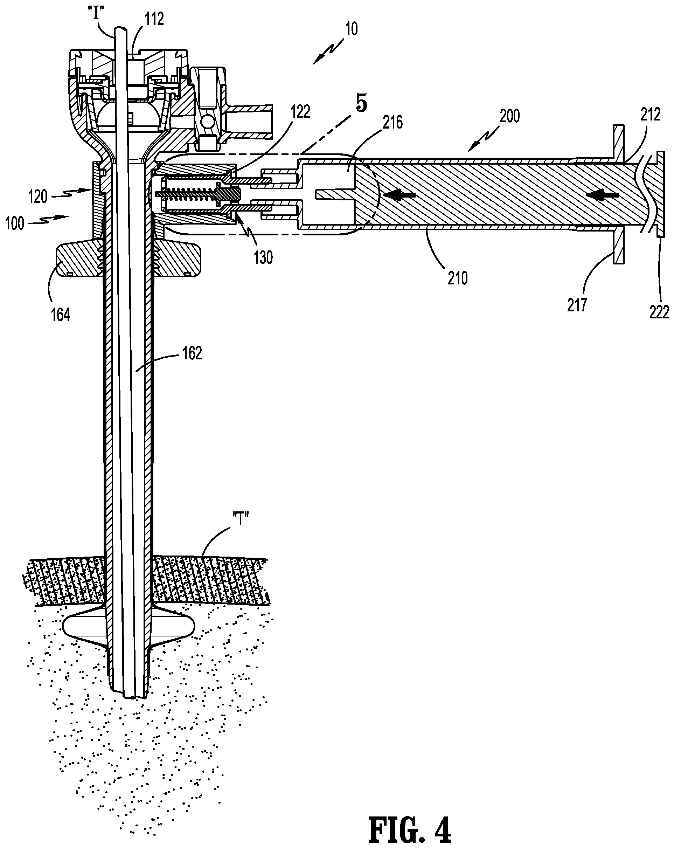

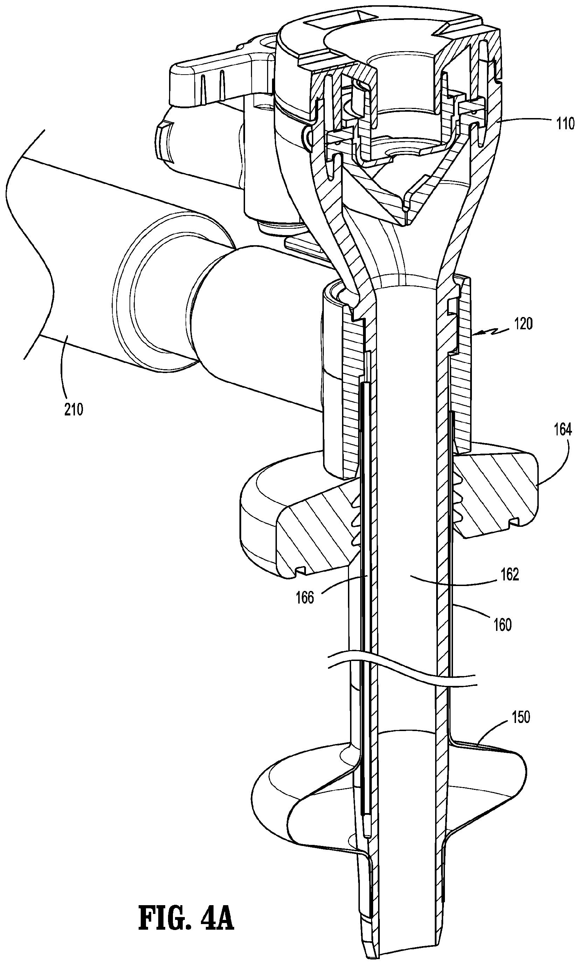

With initial reference to FIG. 1, the presently disclosed surgical access assembly is shown and identified as surgical access assembly 10. The surgical access assembly 10 includes a surgical access device 100 and a syringe 200. The surgical access device 100 has a housing 110 at its proximal end. The housing 110 has an opening 112 that allows insertion of a surgical instrument "I" therethrough (FIG. 4). As such, a clinician is able to perform a surgical procedure at a surgical work site that is below surface tissue "T" (FIG. 4). Examples of surgical instruments include, but are not limited to, graspers, staplers, endoscopes, etc. The housing 110 includes one or more seals (e.g., instrument seal, zero closure seal) that inhibit proximal fluid flow through the housing in the absence of the surgical instrument "I". Additionally, the housing 110 includes an insufflation valve 114. The insufflation valve 114 has a valve handle 116 and an insufflation port 118. The valve handle 116 is rotatable between an open position and a closed position. The insufflation port 118 is attachable to a source of insufflation fluid (e.g., CO₂) (not shown) for insufflating the body cavity thereby separating layers of body tissue and creating a working site.

With additional reference to FIGS. 4 and 4A, the surgical access device 100 also includes an inflation assembly 120 that is disposed in a proximal region of the surgical access device 100 and is located distally of the housing 110. The inflation assembly 120 includes a check valve 130 and a port 122. The port 122 and the check valve 130 of the inflation assembly 120 are in fluid communication with an expandable balloon 150 of the surgical access device 100. A cannula tube 160 extends distally from the housing 110 and supports the expandable balloon 150 in a distal region thereof. The expandable balloon 150 is fluidly coupled to the inflation assembly 120 via a channel 166 that is formed along the cannula tube 160. The channel 166 allows the inflation fluid (e.g., air) to be transferred between the inflation assembly 120 and the expandable balloon 150. The cannula tube includes a lumen 162 extending therethrough that is coincident with the opening 112 of the housing 110. A ring 164 is slidably disposed on the cannula tube 160. The ring 164 frictionally maintains its position along the outer surface of the cannula tube 160 and resists movement along the cannula tube 160 unless the clinician moves the ring 164 along the cannula tube 160. The ring 164 is used to help maintain the position of the surgical access device 100 in body tissue by capturing tissue between the ring 164 and the expandable balloon 150 when the expandable balloon 150 is in the expanded configuration.

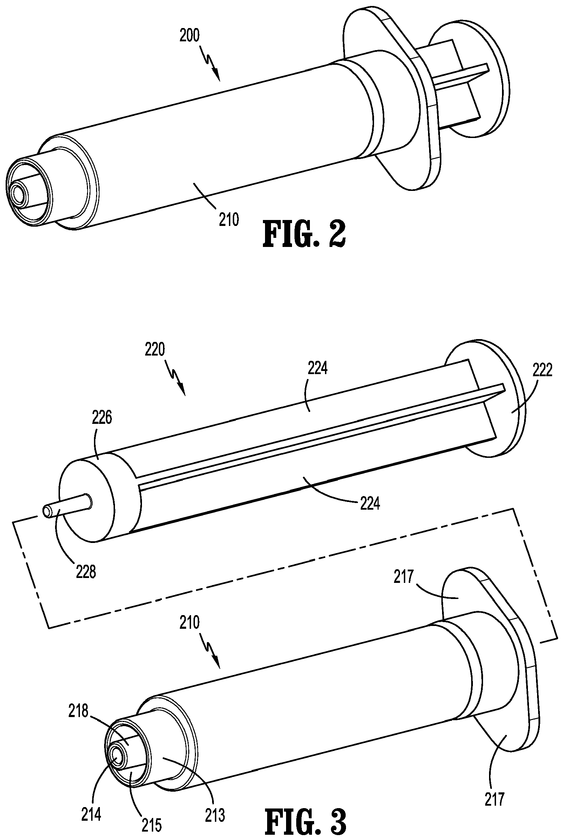

Referring now to FIGS. 2-4, the syringe 200 includes a barrel 210 and a plunger 220. The barrel 210 has proximal and distal openings 212, 214 defining a chamber 216 therebetween. The chamber 216 is configured to slidably receive the plunger 220 therein. The distal opening 214 is located at a distal end of an extension 218 of the barrel 210. The extension 218 has a smaller outside diameter than that of the barrel 210. Additionally, a shroud 213 circumscribes the extension 218 with a diameter greater than that of the extension 218 and less than that of the barrel 210. This arrangement between the shroud 213 and the extension 218 defines a cavity 215 that facilitates coupling the syringe 200 with the inflation assembly 120 of the surgical access device 100. At the proximal end of the barrel are wings 217 configured to facilitate grasping and operating the syringe 200 by the clinician. The plunger 220 has a proximal disc 222 configured to be engaged by finger or thumb of the clinician. The body of the plunger 220 has an X shaped cross-sectional configuration along a majority of its length defined by ribs 224 with a cylindrical section 226 at its distal end. A protrusion or prong 228 extends distally from the cylindrical section 226 and is configured for engaging the check valve 130 as will be described in detail hereinafter. As assembled, the cylindrical section 226 of the plunger 220 is inserted into the chamber 216 through the proximal opening 212. The plunger 220 forms a fluid tight seal between the outer surface of the cylindrical section 226 and the inner surface of the chamber 216 as well as a fluid tight seal between the outer surfaces of the ribs 224 and the inner surface of the chamber 216. As the plunger 220 is translated distally through the chamber 216, air disposed in the chamber is pressurized and discharged through the distal opening 214.

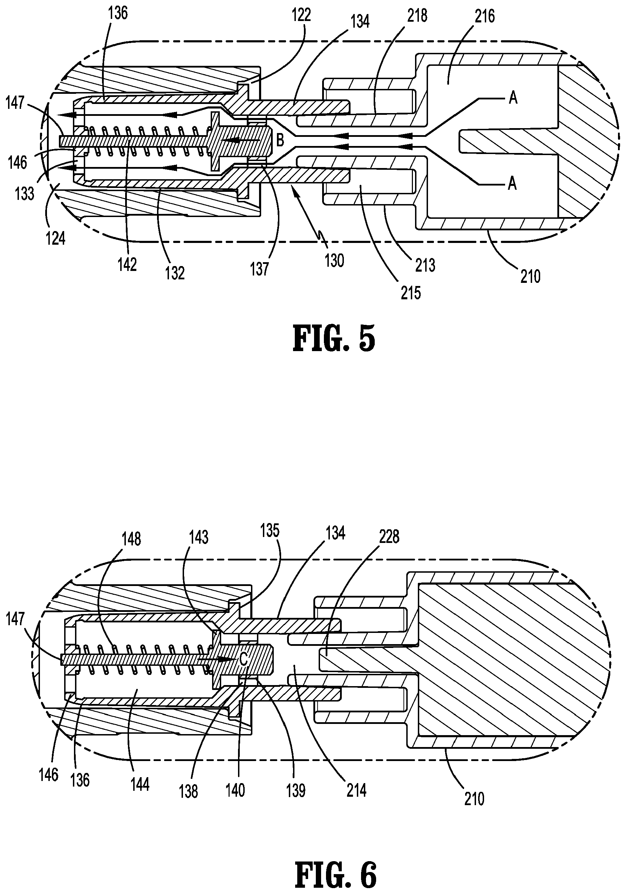

Referring now to FIGS. 4-6, the interaction between the check valve 130 and the syringe 200 will be more fully explained. The check valve 130 is positioned in a passageway 124 of the inflation assembly 120 and extends through the port 122. The check valve 130 has a body 132 with a proximal portion 134 and a distal portion 136. The outer diameter of the proximal portion 134 is less than the outer diameter of the distal portion 136. A rim 135 separates the proximal and distal portions 134, 136 and facilitates securement of the check valve 130 in the passageway 124 of the inflation assembly 120. The rim 135 has a larger diameter than the outer diameter of the proximal and distal portions 134, 136. A proximal disc 138 with an orifice 139 is positioned in the proximal portion 134 and is longitudinally spaced from the rim 135. The orifice 139 is configured to slidably receive a proximal button 140 of a piston 142. The piston 142 is slidable in a cavity 144 of the check valve 130. The piston 142 includes a distally extending rod 147 and a portion of the rod 147 extends through an orifice 145 of a distal disc 146 disposed at the distal end of the distal portion 136. A spring 148 surrounds the rod 147 and is in contact with the distal disc 146 and a flange 143 of the piston 142. The spring 148 biases the piston 142 towards the port 122 in the direction of arrow "C" pressing the flange 143 against the shoulder between the proximal and distal portions 134, 136. This defines the rest or closed configuration of the check valve 130 and inhibits fluid flow proximally towards the port 122 (FIG. 6). The extension 218 of the syringe 200 is insertable into the proximal portion 134 of the check valve 130. When the plunger 220 is translated distally through the chamber 216 of the syringe 200, the inflation fluid (e.g., air) present in the chamber 216 is pressurized and is forced through the extension 218 and out the distal tip 214. The inflation fluid travels through pores 137 of the proximal disc 138 and contacts the proximal surface of the rim 135 and generates a distally applied force. The distally applied force of the inflation fluid overcomes the bias of the spring 148 and urges the piston 142 distally in the direction of arrow "B" such that the inflation fluid can flow through the check valve 130 as indicated by the arrows "A". The inflation fluid travels through the body 132 of the check valve 130 and exits the distal end of the check valve through bores 133 in the distal disc 146. This arrangement defines the actuated configuration of the check valve 130. After passing through the check valve 130, the inflation fluid travels through the channel 166 (FIG. 4A) and into the expandable balloon 150. In sum, when the pressure of the inflation fluid at the port 122 is less than the biasing force of the spring 148, the spring 148 urges the piston 142 towards the port and closes the check valve 130 (i.e., the rest configuration). When the pressure of the inflation fluid at the port 122 is greater than the biasing force of the spring 148, the pressurized inflation fluid urges the piston 142 distally away from the port 122 allowing the inflation fluid to travel through the check valve 130 (i.e., the actuated configuration) and ultimately to the expandable balloon 150.

As the inflation fluid is introduced into the expandable balloon 150, the expandable balloon 150 transitions from a collapsed state to an expanded state. If the volume of inflation fluid in the syringe 200 is insufficient to expand the expandable balloon 150 to a desired size, then the plunger 220 of the syringe is withdrawn out of the chamber 216. This allows air to fill the chamber 216 such that inserting the plunger 220 into the chamber 216 and translating the plunger 220 distally will transfer an additional amount of air into the expandable balloon 150 increasing the size of the expandable balloon 150. Even though the plunger 220 is removed from the syringe 200, the inflation fluid already in the expandable balloon 150 remains there as the check valve 130 is now in its rest configuration. The process of adding inflation fluid may be repeated as desired. Alternatively, the syringe 200 may be separated from the inflation assembly 120 and the plunger 220 partially retracted in the chamber 216 thereby allowing additional air to enter the chamber 216 through the distal opening 214. Subsequently, the syringe 200 is coupled to the inflation assembly 120 as discussed hereinabove and additional inflation fluid is introduced into the expandable balloon 150 as discussed hereinabove.

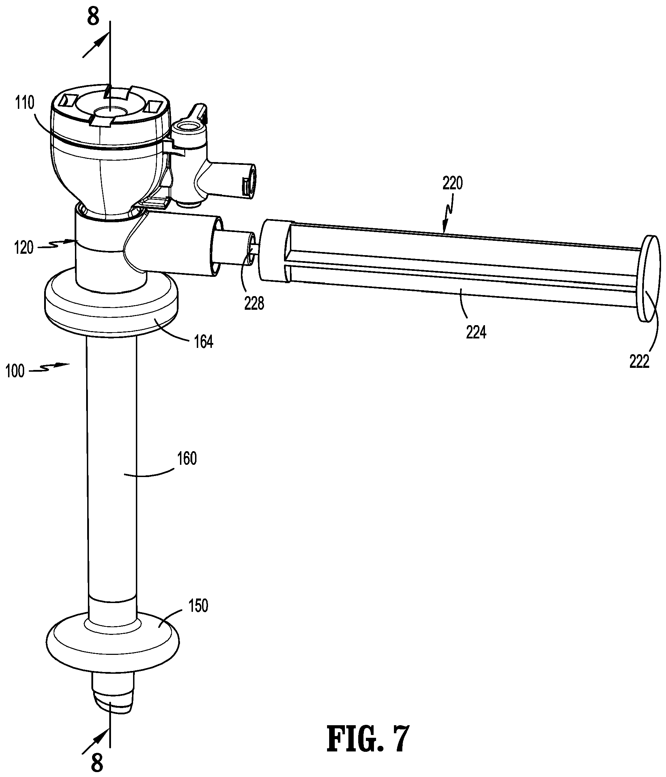

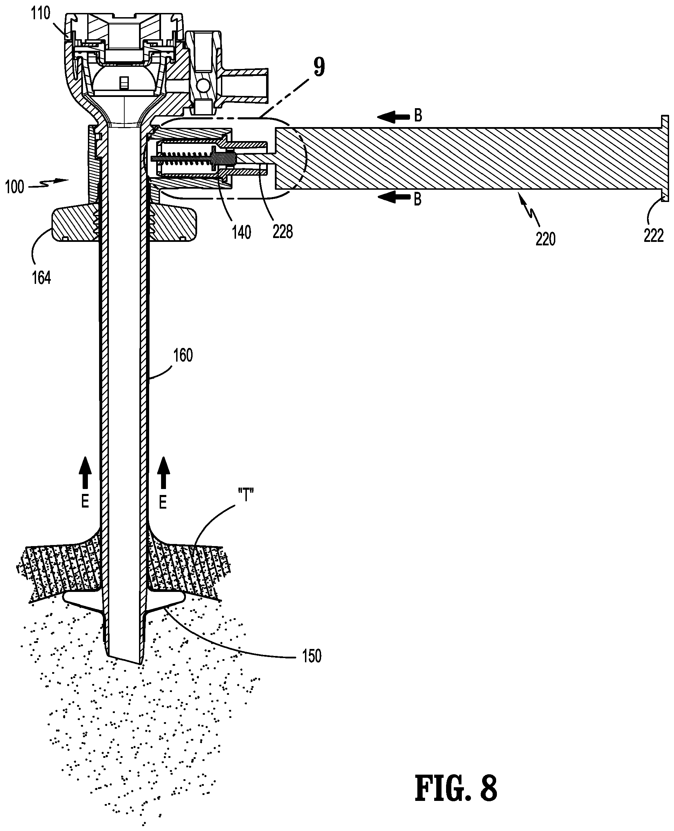

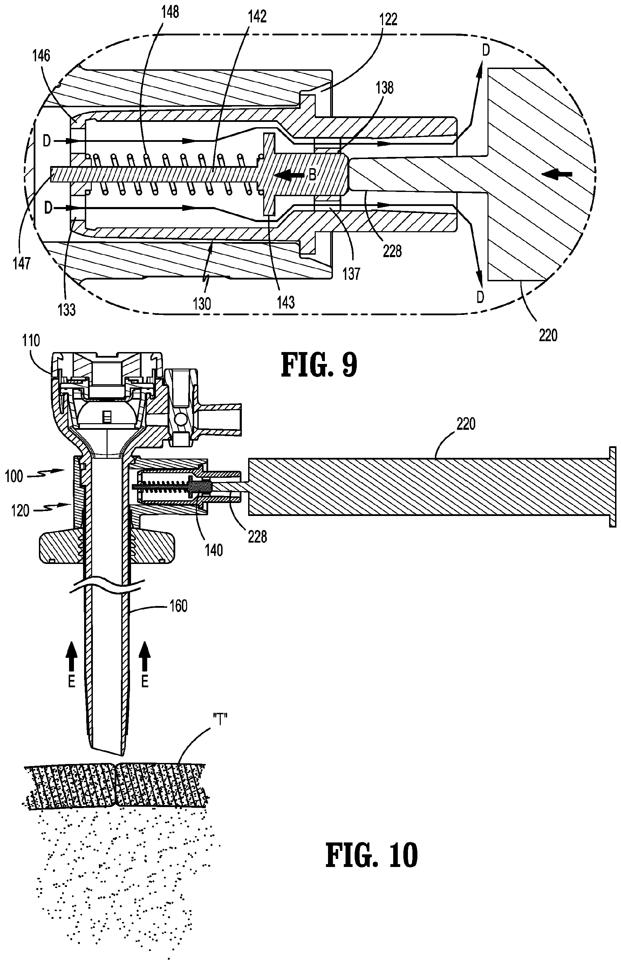

With reference now to FIGS. 7-10, removal of the surgical access device 100 from the work site is facilitated by rapidly collapsing the expandable balloon 150. After the surgical procedure is completed, the clinician inserts the plunger 220 into the inflation assembly 120 such that the prong 228 of the plunger 220 engages the proximal button 140 of the piston 142. Pressing the plunger 220 into the port 122 of the inflation assembly 120 causes the prong 228 to press on the proximal button 140 and overcoming the bias of the spring 148. This translates the piston 142 distally thereby transitioning the check valve 130 from the rest configuration (FIG. 6) to the actuated configuration (FIG. 5), which allows the inflation fluid to flow from the expandable balloon 150 through the channel 166 through the open check valve 130 and out the port 122 of the inflation assembly 120. This causes the expandable balloon 150 to transition towards its collapsed state. In particular, once the prong 228 of the plunger 220 moves the piston 142 distally in the direction of arrow "B", the check valve 130 is now in the actuated configuration. As the pressure of the inflation fluid in the body 132 is greater than the air pressure surrounding the port 122, the inflation fluid travels through the check valve 130 in the direction of arrows "D" exiting the check valve 130. Specifically, the inflation fluid travels through the bores 133 of the distal disc 146, the body 132 of the check valve 130, and the pores 137 in the proximal disc 138. In conjunction with releasing the inflation fluid by manually actuating the check valve 130 with the plunger 220, the clinician may withdraw the surgical access device 100 from the work site. As shown in FIG. 8, withdrawing the surgical access device 100 in the direction of arrows "E" causes the expandable balloon 150 to contact the underside of tissue "T". Continued withdrawal of the surgical access device 100 in combination with venting the inflation fluid through the check valve 130 results in the expandable balloon 150 returning to its collapsed state and easy removal of the surgical access device 100 from the work site (FIG. 10).

Once the clinician identifies the work site, the clinician inserts the cannula tube 160 of the surgical access device 100 through an opening in the tissue "T" (FIG. 4). With the surgical access device 100 in the desired location, the clinician couples the syringe 200 to the port 122 of the inflation assembly 120 and translates the plunger 220 through the chamber 216 of the syringe one or more times to expand the expandable balloon 150 to a desired size such that the expandable balloon 150 anchors the surgical access device 100 at the work site thereby resisting expulsion of the surgical access device 100. Once the surgical access device 100 is anchored, the clinician slides the ring 164 distally until it contacts the outer surface of the tissue "T" helping to maintain the position of the surgical access device 100 relative to the opening. Following the surgical procedure, the clinician inserts the prong 228 of the plunger 220 through the port 122 of the inflation assembly 120 thereby transitioning the check valve 130 from its rest configuration (FIG. 6) to its actuated configuration (FIG. 9) allowing the inflation fluid to escape the expandable balloon 150. Concurrently, the clinician withdraws the surgical access device 100 through the tissue "T" such that the expandable balloon 150 contacts the underside of the tissue "T" which accelerates the transition of the expandable balloon 150 to the collapsed state and facilitates ready removal of the surgical access device 100 from the work site.

The invention may be described by reference to the following numbered paragraphs:
1. A surgical access assembly comprising:
   a surgical access device including
      a cannula tube,
      an expandable balloon positioned in a distal region of the cannula tube, and
      an inflation assembly positioned in a proximal region of the cannula tube, the inflation assembly including a port and a check valve, the port in fluid communication with the expandable balloon, the check valve transitionable between a rest configuration and an actuated configuration, the rest configuration allowing fluid flow in a first direction and inhibiting fluid flow in a second direction opposite the first direction, the actuated configuration allowing fluid flow in the first and second directions; and
   a syringe including
      a barrel having proximal and distal openings defining a chamber therebetween, the chamber configured to store inflation fluid therein,
      an extension configured to releasably engage a portion of the check valve,
      a plunger slidably received in the chamber through the proximal opening and configured to discharge at least a portion of the inflation fluid stored therein through the distal opening, and
      a release feature engageable with the check valve to transition the check valve from the rest configuration to the actuated configuration.
2. The surgical access assembly of paragraph 1, wherein the release feature is insertable into the port to transition the check valve from the rest configuration to the actuated configuration.
3. The surgical access assembly of paragraph 1, further including the distal opening of the barrel engaged with the port such that translation of the plunger into the chamber delivers inflation fluid through the port thereby transitioning the check valve from the rest configuration to the actuated configuration.
4. The surgical access assembly of paragraph 2, wherein the release feature is a protrusion located on a distal end of the plunger.
5. The surgical access assembly of paragraph 3, wherein the expandable balloon transitions from a collapsed configuration to an expanded configuration as the plunger translates distally through the chamber.
6. The surgical access assembly of paragraph 4, wherein the check valve includes a piston and a spring that biases the piston proximally towards the port.
7. The surgical access assembly of paragraph 6, wherein the protrusion is engageable with a portion of the piston and adapted to translate the piston distally thereby transitioning the check valve from the rest configuration to the actuated configuration.
8. A method of accessing a surgical site comprising:
   inserting a portion of a surgical access device into a patient, the surgical access device including a cannula tube, an expandable balloon disposed in a distal region of the cannula tube, and an inflation assembly disposed in a proximal region of the cannula tube;
   coupling a syringe to the inflation assembly such that a distal opening of a barrel of the syringe is positioned in a port of the inflation assembly;
   translating a plunger in a chamber of the barrel thereby pressurizing inflation fluid in the chamber and transitioning a check valve of the inflation assembly from a rest configuration to an actuated configuration such that the chamber is in fluid communication with the expandable balloon; and
   transitioning the expandable balloon from a collapsed configuration to an expanded configuration by continued translation of the plunger towards a distal region of the chamber such that the check valve remains in the actuated configuration and inflation fluid is transferred from the chamber to the expandable balloon.
9. The method of paragraph 8 further including:
   uncoupling the syringe from the inflation assembly;
   inserting a surgical instrument through the cannula tube; and
   performing a surgical procedure at the surgical site.
10. The method of paragraph 9 further including inserting a protrusion of the plunger into the port thereby transitioning the check valve from the rest configuration to the actuated configuration.
11. The method of paragraph 10 further including:
   removing the surgical access device from the patient; and
   transitioning the expandable balloon from the expanded configuration to the collapsed configuration by venting the inflation fluid through the check valve and the port.
12. The method of paragraph 8 further including uncoupling the syringe from the inflation assembly.
13. The method of paragraph 12 further including inserting a protrusion of the plunger into the port thereby transitioning the check valve from the rest configuration to the actuated configuration.

Persons skilled in the art will understand that the devices and methods specifically described herein and illustrated in the accompanying drawings are non-limiting. It is envisioned that the elements, paragraphs and features may be combined with the elements and features of another without departing from the scope of the disclosure. As well, one skilled in the art will appreciate further features and advantages of the disclosure.

## Claims

1. A surgical access assembly (10) comprising:
a surgical access device (100) including
a cannula tube (160),
an expandable balloon (150) positioned in a distal region of the cannula tube, and
an inflation assembly (120) positioned in a proximal region of the cannula tube, the inflation assembly including a port (122) and a check valve (130), the port in fluid communication with the expandable balloon (150), the check valve transitionable between a rest configuration and an actuated configuration, the rest configuration allowing fluid flow in a first direction and inhibiting fluid flow in a second direction opposite the first direction, the actuated configuration allowing fluid flow in the first and second directions; and
a syringe (200) including
a barrel (210) having proximal and distal openings defining a chamber (216) therebetween, the chamber configured to store inflation fluid therein,
an extension configured to releasably engage a portion of the check valve (130),
a plunger (220) slidably received in the chamber through the proximal opening and configured to discharge at least a portion of the inflation fluid stored therein through the distal opening, and
a release feature (228) engageable with the check valve to transition the check valve from the rest configuration to the actuated configuration.

2. The surgical access assembly according to claim 1, wherein the release feature is insertable into the port to transition the check valve from the rest configuration to the actuated configuration.

3. The surgical access assembly according to claims 1 or 2, further including the distal opening of the barrel engaged with the port such that translation of the plunger into the chamber delivers inflation fluid through the port thereby transitioning the check valve from the rest configuration to the actuated configuration.

4. The surgical access assembly according to any of the preceding claims, wherein the release feature is a protrusion located on a distal end of the plunger.

5. The surgical access assembly according to any of the preceding claims, wherein the expandable balloon transitions from a collapsed configuration to an expanded configuration as the plunger translates distally through the chamber.

6. The surgical access assembly according to claim 4, wherein the check valve includes a piston and a spring that biases the piston proximally towards the port.

7. The surgical access assembly according to claim 6, wherein the protrusion is engageable with a portion of the piston and adapted to translate the piston distally thereby transitioning the check valve from the rest configuration to the actuated configuration.

8. A method of accessing a surgical site comprising:
inserting a portion of a surgical access device (I) into a patient, the surgical access device including a cannula tube (160), an expandable balloon (150) disposed in a distal region of the cannula tube, and an inflation assembly (120) disposed in a proximal region of the cannula tube;
coupling a syringe (200) to the inflation assembly such that a distal opening of a barrel (210) of the syringe is positioned in a port (122) of the inflation assembly;
translating a plunger (220) in a chamber (216) of the barrel thereby pressurizing inflation fluid in the chamber and transitioning a check valve (130) of the inflation assembly from a rest configuration to an actuated configuration such that the chamber is in fluid communication with the expandable balloon; and
transitioning the expandable balloon from a collapsed configuration to an expanded configuration by continued translation of the plunger towards a distal region of the chamber such that the check valve remains in the actuated configuration and inflation fluid is transferred from the chamber to the expandable balloon.

9. The method according to claim 8 further including:
uncoupling the syringe from the inflation assembly;
inserting a surgical instrument through the cannula tube for accessing the surgical site.

10. The method according to claims 8 or 9, further including inserting a protrusion of the plunger into the port thereby transitioning the check valve from the rest configuration to the actuated configuration.

11. The method according to any of claims 8 to 10, further including:
removing the surgical access device from the patient; and
transitioning the expandable balloon from the expanded configuration to the collapsed configuration by venting the inflation fluid through the check valve and the port.

12. The method according to any of claims 8 to 11, further including uncoupling the syringe from the inflation assembly.

13. The method according to any of claims 8 to 12 further including inserting a protrusion of the plunger into the port thereby transitioning the check valve from the rest configuration to the actuated configuration.

14. A method of accessing a target site comprising:
inserting a portion of an access device in relation to the target site, the access device including a cannula tube (160), an expandable balloon (150) disposed in a distal region of the cannula tube, and an inflation assembly (120) disposed in a proximal region of the cannula tube;
coupling a syringe (200) to the inflation assembly such that a distal opening of a barrel (210) of the syringe is positioned in a port (122) of the inflation assembly;
translating a plunger (220) in a chamber (216) of the barrel thereby pressurizing inflation fluid in the chamber and transitioning a check valve (130) of the inflation assembly from a rest configuration to an actuated configuration such that the chamber is in fluid communication with the expandable balloon (150); and
transitioning the expandable balloon from a collapsed configuration to an expanded configuration by continued translation of the plunger (220) towards a distal region of the chamber such that the check valve (130) remains in the actuated configuration and inflation fluid is transferred from the chamber to the expandable balloon.

15. The method according to claim 14, further including:
uncoupling the syringe from the inflation assembly;
inserting an instrument through the cannula tube for accessing the target site.
